# EUROPEAN PATENT APPLICATION

(11) **EP 1 050 307 A1**
(43) Date of publication of application: **08.11.2000**
(21) Application number: 99108954.1
(22) Date of filing: 06.05.1999
(51) Int. Cl.: A61K 39/395

(54) **CCR4 antagonists in sepsis**

(71) Applicant: Applied Research Systems ARS Holding N.V., Curacao (AN)
(72) Inventor: Power Christina A., 1288 Aire-la-ville Geneva (CH); Chivatchko Yolande, 1232 Confignon (CH)
(74) Representative: Pieraccioli, Daniele

(57) **Abstract**

CCR4 receptor antagonists are described to be suitable to treat and/or prevent septic shock. The antagonists of this invention typically are selected among several classes but preferably are anti-CCR4 antibodies.

## Description

### FIELD OF INVENTION

CCR4 receptor antagonists can be administered in therapeutically effective doses to treat and/or prevent septic shock. The antagonists of this invention typically are selected among several classes but preferably are anti-CCR4 antibodies.

### BACKGROUND OF INVENTION

Chemokines and their receptors are at the core of many processes in biology, from routine immunosurveillance and the inflammatory process, through to the infection of cells by HIV. In the past two years, various bioinformatic and cloning strategies have led to an explosion in the number of chemokines and receptors that have been identified. Although the picture is far from complete, several themes are emerging.

Chemokines (or chemoattractant cytokines) are a large family of small proteins that are involved both in the routine immunosurveillance that takes place in the body and in the activation and recruitment of specific cell populations during disease. Up until ten years ago, little was known about the proteins that might act as the traffic controllers to recruit specifically leukocyte subpopulations to sites of inflammation. The search for such factors led first to the identification of interleukin 8 (IL-8), a neutrophil chemoattractant, and monocyte chemotactic protein 1 (MCP-1), a monocyte and T-cell chemoattractant. Amino acid sequencing of these chemokines showed two different patterns of four conserved cysteine residues: in IL-8, the two N-terminal cysteines are separated by a single amino acid to form a CXC motif, whereas in MCP-I, they are adjacent, and form a CC motif. These spacings gave rise to the two principal chemokine subclasses, CXC (also known as-chemokines) and CC (also known as -chemokines). Although protein identity levels can be low as 20%, the three-dimensional structures of the monomeric proteins in both groups are almost superimposable.

The first two CXC chemokine receptors identified were found predominantly on neutrophils, and thus it became dogma that CXC chemokines were the drivers of acute inflammation. Much work has focused on their role in diseases such as acute respiratory distress syndrome and septic shock (Folkesson H. G. et al. (1995), J. Clin. Invest., 96:107-116). By contrast, the CC chemokine receptors are expressed on a much wider range of cells, including lymphocytes, monocytes, macrophages, eosinophils, basophils and even platelets, and have been linked to chronic inflammatory diseases such as asthma, arthritis and atherosclerosis. This dichotomy has broken down because the most recently discovered CXC chemokine receptors CXCR3 and CXCR4 are expressed on T cells, and CXCR5 is expressed on B cells. In addition, it has long been known that murine neutrophils can express active CC chemokine receptors, and human neutrophils become responsive to CC chemokines following incubation with interferon (IFN-y) (Bonecchi, R. et al. J. Exp. Med. (in press).

The CC chemokine receptors CCR1-5 bind multiple CC chemokines. As a result of the new chemokines becoming available, the ligand range has also been extended for some receptors. For example, the novel CC chemokines T cell and activation-related chemokine (TARC) and monocyte-derived chemokine (MDC) also bind to CCR4 (Ref. 3), whereas CCR4 was initially described as being activated by macrophage inflammatory protein 1 (MIP-1), RANTES (regulated upon activation normal T expressed and secreted) and monocyte chemoattractant protein 1 (MCP-1) CXCR4, CCR6 and CX3CR1 remain highly selective, only binding to one chemokine out of 30 tested in most cases. It remains to be seen if this apparent selectivity holds out as new chemokines are discovered. The idea that restrictive expression of the receptor is associated with a restrictive ligand-binding pattern is attractive, but this might merely be an artifact of the recent discovery.

The role of chemokines in inflammation has also been validated by the use of monoclonal antibodies in inflammatory models: MIP-1 antibodies significantly reduce eosinophilia in the S. mansoni egg antigen model (Standiford T. J. et al. (1995) J. Immunol., 155:1515-1524); antibodies to IL-8 prevented neutrophil-mediated sepsis in the rabbit (Folkesson H. G. et al. (1995), J. Clin. Invest., 96:107-116; Yokoi K. I. et al. (1997) Lab. Invest., 76:375-384); and anti-MCP-1 antibody significantly reduced cellular recruitment in glomerulonephritis (Lloyd C. M. et al. (1997) J. Exp. Med., 185:1371-1380) and granuloma models (Flory C. M. et al. (1993) Lab. Invest., 69:396-404). These results confirm that despite the apparent complexity of the system, elimination of a single chemokine or receptor can significantly alter models of pathology.

### DESCRIPTION OF INVENTION

We have now found that CCR4 plays a role in sepsis and that CCR4 receptor antagonists can be administered in therapeutically effective doses to treat and/or prevent septic shock. The antagonists of this invention typically are selected among several classes but preferably are anti-CCR4 antibodies.

Therefore, the main object of the present invention is to provide a method to treat and/or prevent septic shock in an individual comprising administering a therapeutically effective amount of CCR4 antagonist.

A still further object of the present invention is the use of a CCR4 antagonist together with a pharmaceutically acceptable carrier in the preparation of pharmaceutical compositions for treatment of septic shock. The pharmaceutical compositions prepared in this way are also a further object of the present invention.

"Pharmaceutically acceptable" is meant to encompass any carrier, which does not interfere with the effectiveness of the biological activity of the active ingredient and that is not toxic to the host to which is administered. For example, for parenteral administration, the above active ingredients may be formulated in unit dosage form for injection in vehicles such as saline, dextrose solution, serum albumin and Ringer's solution. Besides the pharmaceutically acceptable carrier, the compositions of the invention can also comprise minor amounts of additives, such as stabilizers, excipients, buffers and preservatives.

The administration of such active ingredient may be by intravenous, intramuscular or subcutaneous route. Other routes of administration, which may establish the desired blood levels of the respective ingredients, are comprised by the present invention.

The active ingredients of the claimed compositions herein are CCR4 antagonists. Preferably, they are polypeptides that bind CCR4 with high affinity. More preferably, they are anti-CCR4 antibodies. They can be prepared as specifically described in WO 96/23068.

As used herein, the term "antibodies" includes polyclonal antibodies, monoclonal antibodies, antigen-binding fragments hereof such as F(ab')₂ and Fab fragments, and the like, including genetically engineered antibodies. Antibodies are defined to be specifically binding if they bind to a CCR4 polypeptide with a K_{d} of greater than or equal to 10⁷/M. The affinity of an antibody can be readily determined by one of ordinary skill in the art (see, for example, Roit, Essential Immunology, fifth ed.. Blackwell Scientific Publications, 1984).

Methods for preparing polyclonal and monoclonal antibodies are well known in the art (see, for example, Sambrook et al., Molecular Cloning: A Laboratory- Manual. second Ed.. Cold Spring harbor, NY, 1989; and Hurrel, Ed., Monoclonal Hybridoma Antibodies: Techniques and Applications. CRC Press, Inc., Boca Raton, FL, 1982). As would be evident to one of ordinary skill in the art, polyclonal antibodies can be generated from a variety of warmblooded animals such as horses, cows, goats, sheep, dogs, chickens, rabbits, mice, and rats. The immunogenicity of a peptide or polypeptide may be increased through the use of an adjuvant such as Freund's complete or incomplete adjuvant. A variety of assays known to those skilled in the art can be utilized to detect antibodies which specifically bind to polypeptides (see Harlow and Lane (Eds.), Antibodies: A Laboratory Manual. Cold Spring Harbor Laboratory Press, 1988).

Monoclonal antibodies may be obtained by well-established methods, e.g. as described in A. Johnstone and R. Thorpe, Immunochemistry in practice. 2nd. Ed., Blackwell Scientific Publications, 1987, pp. 35-43.
Generally, monoclonal antibodies are produced by immunizing an animal with a biological specimen or other foreign substance, obtaining antibody-producing cells from the animal, and fusing the antibody-producing cells with strains of neoplastic cells, e.g. tumor cells, to produce hybridomas which are isolated and cultured as monoclones. The monoclonal hybridomas may either be cultured *in vitro* or may be grown *in vivo* as tumors in a host animal. Because each antibody-producing cell line produces a single unique antibody, the monoclonal cultures of hybridomas each produce a homogenous antibody population which may be obtained either from the culture medium of hybridoma cultures grown *in vitro* or from the ascitic fluid, or serum of a tumor-bearing host animal. Not all of the clones which result from fusion of neoplastic cells with antibody-producing cells are specific for the desired foreign substance or antigen, because many of the hybridomas will secrete antibodies which the animal's immune system has generated in reaction to other foreign substances. Even monoclonal antibodies against the subject antigen will differ from clone to clone because antibodies produced by different clones may react with different antigenic determinants of the same molecule. From each clone, therefore, it is necessary to obtain the resulting antibody or the antibody-containing medium, serum or ascitic fluid and test its reactivity with the subject biological material and to test its specificity by determining what other biological material, if any, it recognizes.

When prepared by recombinant DNA techniques, the antibody may be produced by cloning a DNA sequence coding for the antibody or a fragment thereof into a suitable cell, e.g. a microbial, plant, animal or human cell, and culturing the cell under conditions conducive to the production of the antibody or fragment in question and recovering the antibody or fragment thereof from the culture. Possible strategies for the preparation of cloned antibodies are discussed in, for instance, L. Riechmann et al., Nature 332, 24 March 1988, p. 323 ff., describing the preparation of chimeric antibodies of rat variable regions and human constant regions; M. Better et al., Science 240, 20 May 1988, p. 1041 ff., describing the preparation of chimeric mouse-human Fab fragments; A. Sharra and A. Plückthun, Science 240, 20 May 1988, pp. 1038-1040, describing the cloning of an immunoglobulin Fv fragment containing antigen-binding variable domains; and E.S. Ward et al., Nature 341, 12 October 1989, pp. 544-546, describing the cloning of isolated antigen-binding variable domains ("single-domain antibodies"). (Humanized monoclonal antibodies in general see, for example, Molecular Biology and Biotechnology (3rd ed.), Walker and Gingold (eds.), The Royal Society of Chemistry 1993, p 357-385).

Monoclonal antibodies or other genetically engineered antibodies with specificity for the CCR4 polypeptide could be used as therapeutic agents. The antibodies should then be humanized to reduce the immunogenicity.
Humanization is done by grafting the Complementary-Determining Region (CDR) from the original murine antibody to the constant regions of a human antibody. Various methods can he used to ensure the specificity and avidity of the grafted antibody (Queen,C et al, Proc. Natl. Acad. Sci. U.S.A., 86, 10029, 1989 & Reichmann, L. et al, Nature,332, 323, 1988).

Antibodies to CCR4 polypeptide may be used for isolation, for affinity purification, for diagnostic assays, for determination of circulating levels of CCR4 polypeptides, and as antagonists to block CCR4 activity *in vitro and in vivo.* (See, for example, Immobilized Affinity Ligand Techniques. Hermanson et al., eds., Academic Press, San Diego, CA, 1992, pp. 195-202).

CCR4 antagonist can be administered to an individual in a variety of ways. The routes of administration include intradermal, transdermal (e.g. in slow release formulations), intramuscular, intraperitoneal, intravenous, subcutaneous, oral, epidural, topical, and intranasal routes. Any other therapeutically efficacious route of administration can be used, for example absorption through epithelial or endothelial tissues or by gene therapy wherein a DNA molecule encoding the CCR4 antagonist is administered to the patient (e.g. via a vector) which causes the CCR4 antagonist to be expressed and secreted *in vivo.* In addition the CCR4 antagonist can be administered together with other components of biologically active agents such as pharmaceutically acceptable surfactants, excipients, carriers, diluents and vehicles.

For parenteral (e.g. intravenous, subcutaneous, intramuscular) administration, CCR4 antagonists can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The therapeutically effective amounts of CCR4 antagonist will be a function of many variables, including the type of antagonist, the affinity of the antagonist for CCR4, any residual cytotoxic activity exhibited by competitive antagonists, the route of administration, the clinical condition of the patient (including the desirability of maintaining a non-toxic level of endogenous CCR4 activity). A "therapeutically effective amount" is such that when administered, the CCR4 antagonist results in inhibition of the biological activity of CCR4. The dosage administered, as single or multiple doses, to an individual will vary depending upon a variety of factor, including CCR4 antagonist pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled, as well as *in vitro* and *in vivo* methods of determining the inhibition of CCR4 in an individual.

Usually a daily dosage of active ingredient can be about 0.01 to 100 milligrams per kilogram of body weight. Ordinarily 1 to 100 milligrams per kilogram per day given in divided doses or in sustained release form is effective to obtain the desired results. Second or subsequent administrations can be performed at a dosage which is the same, less than or greater than the initial or previous dose administered to the individual. A second or subsequent administrations can be administered during or prior to relapse of the septic shock or the related symptoms. The terms "relapse" or "reoccurrence" are defined to encompass the appearance of one or more of symptoms of septic shock.

The present invention will now be illustrated by the following Examples, which are not intended to be limiting in any way, and makes reference to the following Figures.

### DESCRIPTION OF THE FIGURES

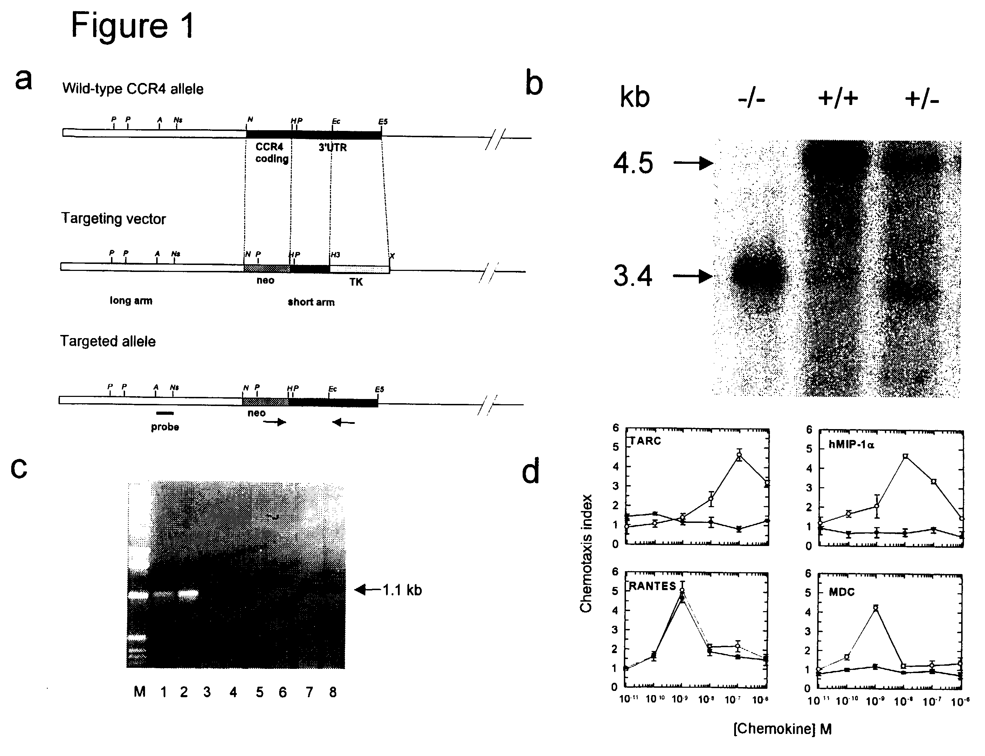

**Figure 1**. Targeted disruption of the CCR4 gene. (a) Targeting strategy. Wild type CCR4 locus with partial restriction map (top), targeting vector (middle) and predicted structure of the targeted allele after homologous recombination (bottom). The coding region of the gene is shown as a black box. The neomycin resistance gene is dark grey and thymidine kinase gene is light grey. The arrows denote the position of the PCR primers used to identify ES cell clones expressing the transgene. The probe used for screening genomic DNA is shown by the thick black bar (probe). Restriction sites: P, PstI; N, NheI; Ns, NsiI; A, AvrI; X, XhoI; E5, EcoRV; Ec, Eco47-3; H, HpaI; H3, HindIII. (b) Representative Southern blot analysis of Pst1 digested tail DNA from wild type (+/+), heterozygote (+/-) and homozygous knockout mice (-/-). The expected bands sizes of the wild type allele (4.5 kb) and the targeted allele (3.4kb) are indicated by arrows. (c) RT-PCR analysis of chemokine receptor mRNA in spleen and thymus of *CCR4*+/+ and -/- mice. (d) Chemotaxis of splenocytes isolated from *CCR4*+/+ (circles) and -/- mice (squares) in response to CCR4 ligands.

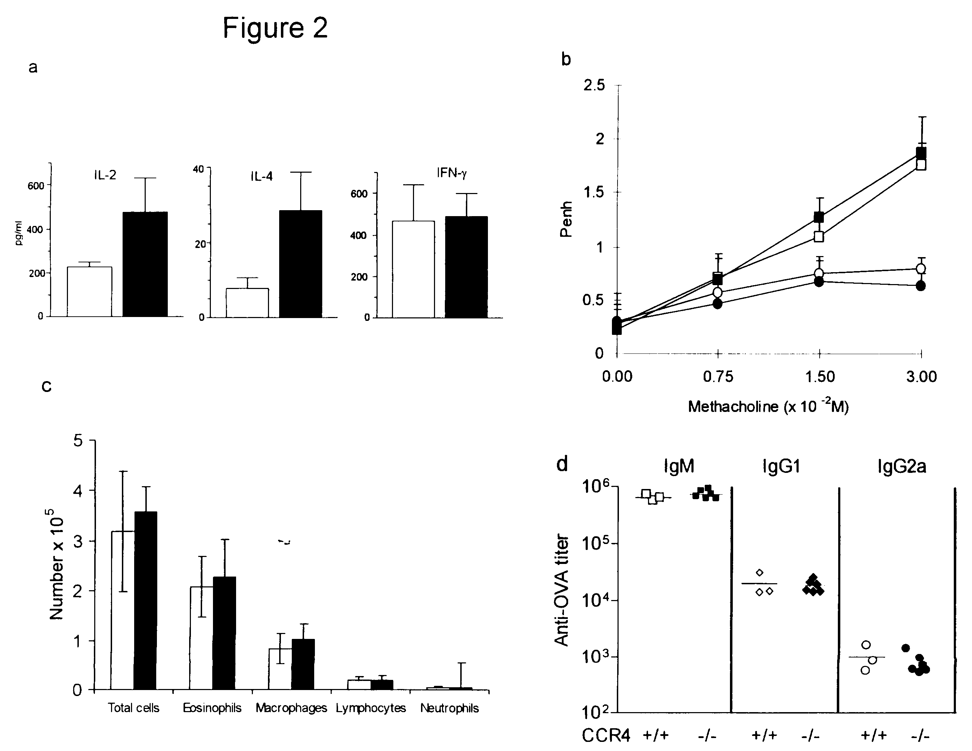

**Figure 2**. (a) Cytokine production by ConA stimulated naïve splenocytes from CCR4 -/- mice (white bars) and CCR4+/+ mice (black bars). Spleens from naïve mice were dispersed in culture (10⁶ cells/ml) and stimulated with 5 µg/ml ConA. Cytokine levels were measured by ELISA 24h later (b) CCR4 deficient mice develop allergic airways inflammation. Increased airway responsiveness to methacholine was measured by whole body plethysmography⁹. This method allows measurements of spontaneous breathing in a non-anaesthetized mouse by recording respiratory pressure curves before and after methacholine inhalation. From the curves, values for the enhanced pause (Penh) are calculated and used as an index of bronchial hyperresponsiveness (BHR). BHR in *CCR4*+/+ mice (open symbols) and *CCR4*-/- (closed symbols) mice after priming with 10 µg of OVA in 0.2 ml of alum and followed by intra-nasal challenge with either 50 µl of 0.9 % NaCI (circles n=10) or OVA (0.3 mg/ml) (squares n=13). (c) Total cell count and individual leukocyte populations (eosinophils, macrophages, lymphocytes and neutrophils) in broncho-alveolar lavage (BAL) fluid. *CCR4*+/+ (open bars n=13) and CCR4 (-/-) (black bars n=13) 72 h after the last OVA-challenge. (d) OVA-specific Ig titers in the sera of OVA-primed and challenged mice. *CCR4*+/+ (open symbols) and *CCR4*-/- (closed symbols). Blood was sampled 72 h after the final intra-nasal OVA-challenge and was tested for the presence of anti-OVA IgM (squares), IgG1 (diamonds), IgG2a (circles) by ELISA¹⁰. Data is shown from one experiment representative of at least three different experiments for each parameter measured.

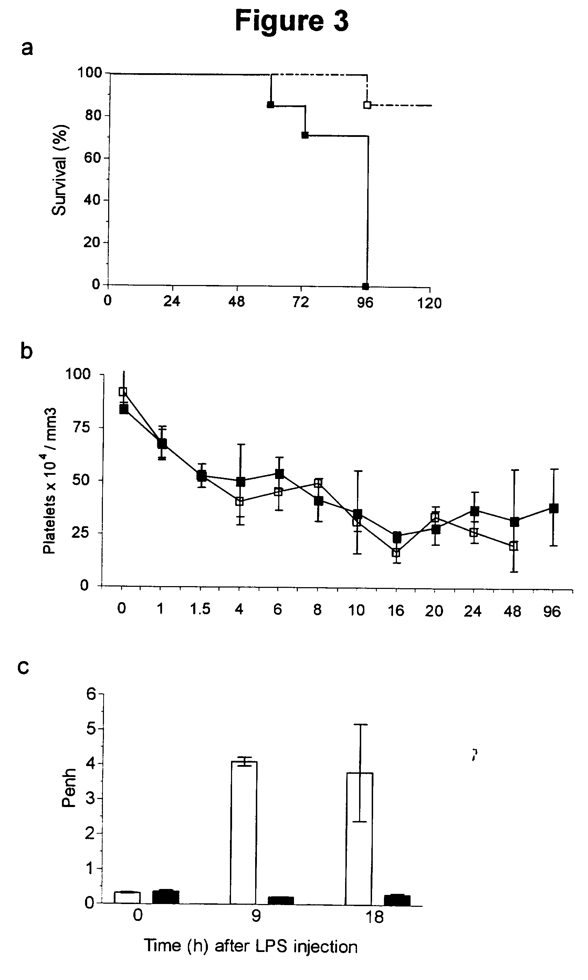

**Figure 3**. Absence of CCR4 protects against LPS-induced sepsis. (a) Survival curves of *CCR4*+/+ and CCR4-/- mice injected intra-peritoneally with 60 mg/kg LPS. The data shown represents a study using 8 animals per group. (b) LPS induced thrombocytopenia . Platelet counts were done on at least 3 animals per group at each time point up to 48h.

Results shown are representative of at least 2 different experiments. (c) Bronchial hyperreactivity after intra-peritoneal injection of LPS. Results shown are means ± s.d. of 6 *CCR4*+/+ and 8 *CCR4*-/- mice per group representative of at least two experiments.

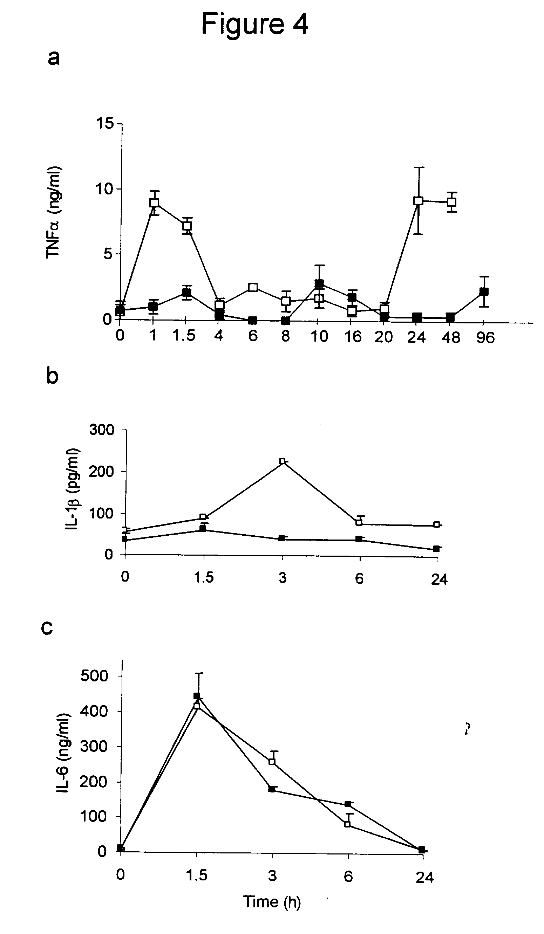

**Figure 4**. Determination of serum TNFα (α), IL-1β (b), and IL-6 (c) in *CCR4*+/+ mice (diamonds) and *CCR4*-/- mice (squares) after LPS treatment. Cytokines were measured by ELISA using kits purchased from R&D systems. Results shown are the means ± s.d of 3 animals per group representative of at least 2 different experiments.

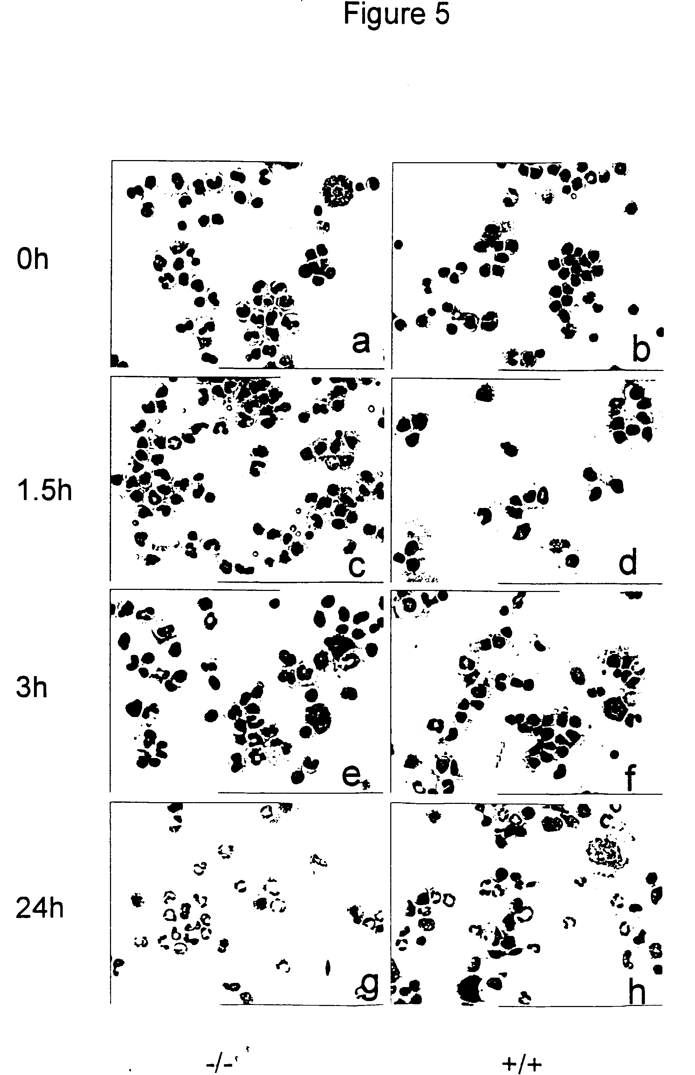

**Figure 5**. Analysis of peritoneal lavage cells by cytospin. 8-12 week old mice were injected with LPS and killed by CO2 asphyxiation 0, 1.5 , 3 and 24 h later. Peritoneal cells were recovered by lavage with 0.9% NaCI, counted, and 5 x 10⁴ cells were cytospun and stained with Diff-Quik.

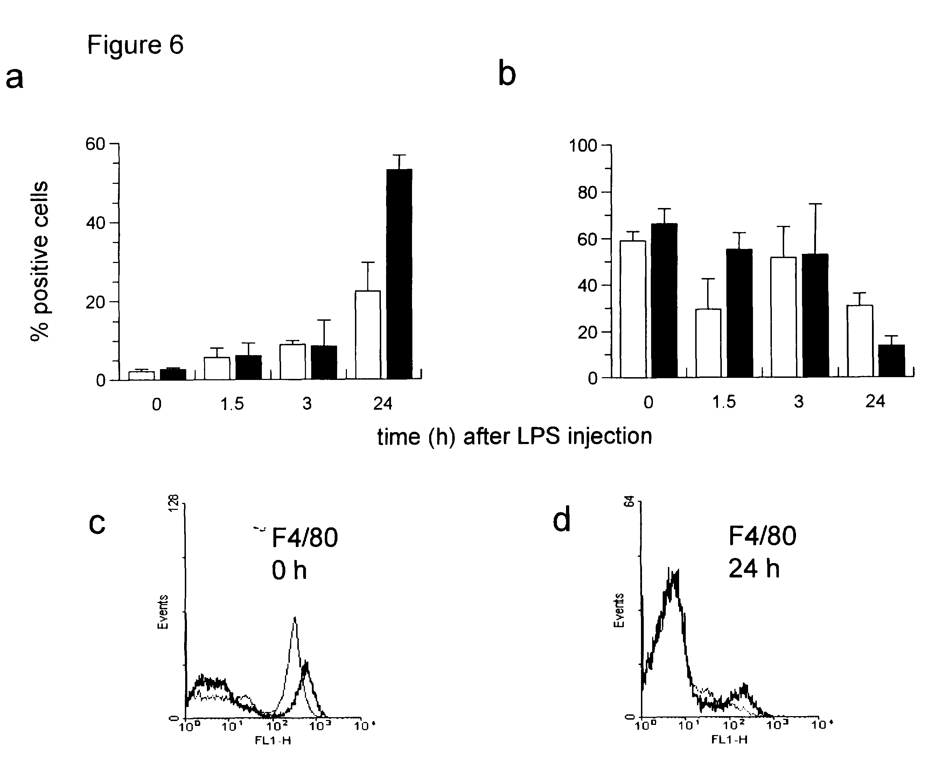

**Figure 6**. FACs analysis of peritoneal lavage cells after LPS treatment. Lavage cells were harvested as described in Fig. 5 and resuspended at 10⁶/ml in FACs buffer. Cells were first incubated with Fc block (Pharmingen) for 10 min at 4 C, washed twice with FACs buffer and then incubated for 20 min with FITC labelled F4/80 (Serotec) or PE labelled GR1 (Pharmingen). Cells were washed twice, resuspended in 200 µl FACS buffer and analyzed on a Becton Dickinson FACstar. F4/80 (a,c,d); GR1 (b)

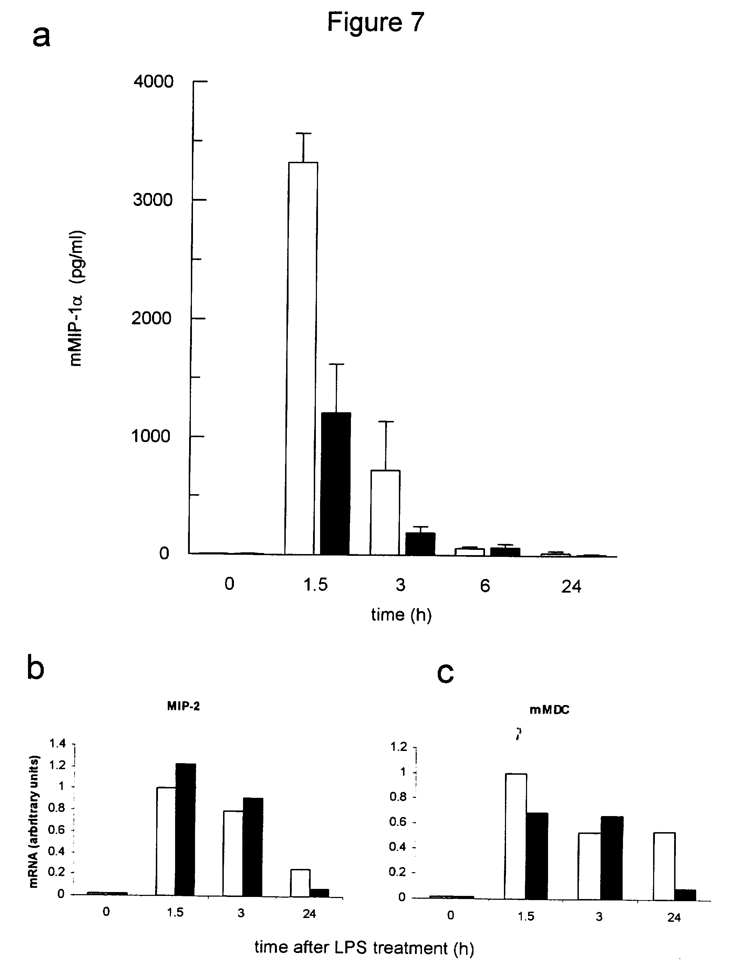

**Figure 7**. Effect of LPS treatment on macrophage chemokine expression. (a) Serum MIP-1α was measured by ELISA using an R&D systems kit. (b) MIP-2 and MDC mRNA was measured by semi-quantitative RT-PCR in peritoneal lavage cells.

### EXAMPLES

### CCR4 Knockout mice

The murine CCR4 gene was deleted through homologous recombination using the targeting vector shown in fig. 1a. Targeted ES cells were used to generate chimeric mice which transmitted the transgene through the germ line. Southern blot analysis confirmed that the CCR4 gene had been targeted (fig. 1b). CCR4 knockout mice were viable, appeared to develop normally, and showed no overt morphological or behavioural defects in the unstressed state. Normally, CCR4 mRNA is expressed in T cells, predominantly in the thymus, spleen and peripheral blood (Power C.A. et al., (1995), J. Biol. Chem. 270, 19495-19500; Hoogewerf A.J. et al., (1996), Biochem. Biophys. Res. Commun. 218, 337-343). We used reverse transcriptase-PCR to demonstrate that the mRNA for CCR4 was not present in the targeted animals (Figure 1c).

### Methods

The murine CCR4 gene was isolated from an HM-1 embryonic stem cell library in λFIXII vector (Stratagene) by plaque hybridisation using murine CCR4 cDNA as a probe (Hoogewerf A.J. et al., (1996), Biochem. Biophys. Res. Commun. 218, 337-343). Two unique clones of 12.18 kb and 13.08 kb were shown to contain the mCCR4 coding sequence by PCR, using specific primers. An 8.5 kb fragment of genomic DNA identified to contain the CCR4 coding sequence by Southern blotting was subcloned into pBluescript II SK- to generate pCCR4. The entire CCR4 coding sequence was then removed as an NheI/Hpal fragment and replaced with a neo cassette (derived from plasmid pMClneoPolyA (Clontech). The resultant construct was digested EcoRV and Eco47-3, and religated, to generate a plasmid containing a long arm of homology of 4904 bp and a short arm of homology of 1318 bp. Finally, a thymidine kinase (tk) cassette was inserted into the HindIII/XhoI site of the plasmid to produce the targeting vector. The targeting vector was linearized with NotI and electroporated into HM-1 embryonic stem cells as described previously (Conquet F. et al., (1994), Nature 372, 237-243). Gancyclovir and G418 resistant clones were selected. DNA was isolated from resistant clones using DNAzol (Gibco-BRL), and the presence of the transgene was detected by PCR, and verified by Southern hybridization following PstI digestion of genomic DNA using a 466 bp probe derived by AvrII/NsiI digestion of pCCR4. Seven independent transgene-containing ES cells were used to produce chimeric mice by blastocyst injection according to standard procedures (McMahon A.P and Bradley A. (1990). Two 100% chimeric females were mated with a 100% chimeric male to generate heterozygous CCR4 (+/-) mice, and littermates from the matings of heterozygous mice were analyzed for the presence of homozygous CCR4 (-/-) knockout mice by southern blot analysis or by PCR on tail DNA.
Chemotaxis assays were performed using the micro Boyden chamber method (Bacon et.al). Recombinant human and mouse chemokines were purchased from R&D systems.

### CCR4 as a physiological receptor of MIP-1α

Although originally identified as a receptor for MIP-1α and RANTES, CCR4 is in fact a high affinity receptor for two recently described chemokines, thymus and activation regulated chemokine (TARC) (Imai T. et al., (1997), J. Biol. Chem. 272, 15036-15042) and macrophage derived chemokine (MDC) (Imai T., et al. (1998), J. Biol. Chem. 273, 1764-1768). We therefore looked at the ability of splenocytes and thymocytes isolated from the targeted and wild type mice to migrate in response to the proposed CCR4 ligands. Splenocytes from *CCR4*-/- mice had no chemotactic response to TARC or MDC whereas splenocytes from *CCR4*+/+ mice responded with the characteristic dose-response curve (fig. 1d), confirming that the gene deleted in this study is an endogenous TARC and MDC receptor. However, splenocytes isolated from the CCR4-/- mice responded neither to human MIP-1α nor to murine MIP-1α (data not shown). This was surprising since the response to RANTES was similar in both the CCR4+/+ and -/- mice. All of the RANTES receptors described to date (CCR1, CCR5, and CCR3 in mouse) have also been shown to bind and signal in response to MIP-1α *in vitro.* RT-PCR analysis of the cell populations used in the study confirmed that deletion of the CCR4 gene did not interfere with expression of these receptors at the mRNA level (data not shown). Taken together these results indicate that under these conditions, CCR4 is a physiological receptor for MIP-1α.

### CCR4 deletion in a Th2 type disease

Increasing numbers of reports show that CCR4 is highly expressed in human Th2 polarized cells (Sallusto F. et al, (1998), J. Exp. Med. 187, 875-883; Bonecchi R. et al, (1998) J. Exp. Med. 187, 129-134; D'Ambrosio D. et al., 1998; and Imai T. et al., 1999), which are also responsive to the CCR4 ligands TARC and MDC, suggesting a possible role for this receptor in the development of Th2 responses. However at present, it is unclear if this paradigm can be applied to murine T cells. We therefore looked at the ability of naïve splenocytes from wild type and CCR4 knockout mice to produce Th1 and Th2 cytokines in response to concanavalin A (ConA), a potent polyclonal activator of T cells. Splenocytes from *CCR4*-/- produced comparable levels of IL-2 and IFN-γ to *CCR4*+/+ mice and slightly elevated levels of the Th2 cytokine IL-4, indicating that the cells from *CCR4*-/- mice were not generally defective in the production of these cytokines (figure 2a). We then studied the effect of the CCR4 deletion in an ovalbumin-induced murine model of airways inflammation, a predominantly Th2 type disease.

Repeated intranasal ovalbumin (OVA) challenges in immunised *CCR4*-/- and +/+ littermates resulted in a significant increase in bronchial hyperreactivity to inhaled methacholine (Hamelmann E. et al., (1997), Am. J. Respir. Crit. Care Med. 156, 766-775) in both groups of animals when compared to saline-challenged mice (Fig. 2b). Penh values were 0.8 ± 0.10 and 1.77 ± 0.2 in saline and OVA-challenged *CCR4*+/+ mice respectively and 0.64 ± 0.11 and 1.88 ± 0.33 in saline-and OVA-challenged *CCR4*-/- mice respectively. Comparable ovalbumin-induced eosinophilia was observed in *CCR4*+/+ and -/- littermates (Fig.2c), a finding consistent with the selective induction of a Th2 response in the airways (Chvatchko Y. et al, (1996), J. Exp. Med. 184, 2353-2360). No significant differences were observed in the broncho-alveolar lavage fluid in either the total cell count, or in other leukocyte populations (macrophages, lymphocytes and neutrophils) between OVA-challenged *CCR4*+/+ and -/- littermates (Fig. 2c). To confirm that efficient antigen priming had occurred in the periphery, serum titers of OVA-specific IgM, IgG1 and IgE in OVA-sensitised and challenged *CCR4*+/+ and -/- littermates were analysed (Blyth D.I. et al., (1996) Am. J. Resp. Cell Mol. Biol. 14, 425-438). Again, OVA-specific IgM, IgG1 and IgE titers were comparable in *CCR4*+/+ and -/- littermates (Fig. 2d). Taken together, these results suggest that deletion of the CCR4 gene does not impair the development of a Th2 response *in vivo.*

### CCR4 knockout mice in LPS-induced septic shock

As CCR4 is also expressed on other cell types, for example monocyte/macrophages and platelets (Power C.A. et al, (1995) Cytokine 7, 479-482 and K. Clemetson et al., submitted), we next looked at the effect of the CCR4 knockout in a distinct model of inflammation in which these cell types are implicated, LPS-induced septic shock (Pajkrt D. et al., (1996) Curr. Topics Microbiol. Immunol 216, 119-132; Freudenberg et al., 1986). CCR4 (+/+) and (-/-) littermates were injected intra-peritoneally with LPS (60 mg/kg). Wild-type mice exhibited a markedly higher rate of mortality compared to CCR4 (-/-) mice (Fig. 3a). CCR4 (+/+) mice died within 96 h of LPS injection whereas over the same period, only 20 % of the CCR4 (-/-) mice died. Nevertheless, *CCR4-/-* mice still showed signs of endotoxaemia such as shivering and lethargy, a few hours after LPS administration, although these effects were clearly milder than in the *CCR4*+/+ mice.
Intra-peritoneal injection of LPS is usually followed by a marked thrombocytopenia (Shibazaki M. et al. (1996) Infection and Immunity 64, 5290-5294), and accumulation of platelets in the lungs, liver and spleen. The blood of *CCR4*-/- contained similar numbers of platelets to *CCR4*+/+ mice. Furthermore, we observed a parallel decrease in blood platelet count in both groups of mice in the first 20 h after LPS injection (Fig. 3b) which then appeared to return to normal in the *CCR4*-/- mice, indicating that there was no obvious difference in the platelet mobilisation between the two groups of mice.
The presence of bacterial products such as LPS in the blood stream is circulatory collapse and severe hypotension, which are associated with potentially lethal conditions including acute lung injury. LPS administration leads to an increase in the vascular permeability in the lungs (Standiford T.J.et al., (1995) J.Immunol. 155, 1515-1524). As a consequence of this, mice develop bronchial hyperreactivity (Y. Chvatchko et al., manuscript in preparation). Bronchial hyperreactivity was observed only in the *CCR4*+/+ mice with peaks at 9 h and 18 h after LPS injection, (Fig. 3c). *CCR4*-/- mice had little or no bronchial hyperreactivity. LPS stimulates the release of inflammatory cytokines such as TNFα and IL-1β from monocyte/macrophages and neutrophils. Here, we have shown that the LPS-induced pulmonary response is preceded by the production of TNFα. Interestingly, *CCR4*-/- mice failed to release significant levels of TNFα in response to LPS injection compared to *CCR4*+/+ mice (Fig. 4a). This suggests that the observed resistance to LPS may be in part due to decreased TNFα implying that CCR4 may be indirectly involved in the regulation of TNFα. In addition to TNFα, we also observed decreased IL-1β in serum (Fig 3d). The production of IL-6 (mainly by hepatocytes) occurs after gram-negative bacterial infection or TNFα infusion (Akira S. et al., (1993), Adv. Immunol. 54, 1-78). We observed no difference in IL-6 production between *CCR4*+/+ and -/- mice (Fig. 4c) suggesting that in the *CCR4*-/- mice the regulation of TNFα and IL-1β is independent from that of IL-6.
We next looked at the cellular composition of the peritoneal lavage at various times after LPS injection by cytospin analysis. No major differences were seen in the numbers and types of leukocytes recruited at early time points (Figs. 5 a-f). However, 24 h after LPS treatment, the leukocyte population in the peritoneal lavage of *CCR4*-/- mice was almost entirely composed of neutrophils (>80%) with few macrophages and no lymphocytes detectable (Fig. 5g) whereas in *CCR4*+/+ mice, macrophages still comprised nearly 70% of the lavage cells, although the number of neutrophils was significantly increased from that seen at earlier time points (Fig. 5h). The enhanced neutrophil recruitment seen in the *CCR4*-/- mice may be in response to a defect in macrophage recruitment (see below). At 1.5 h and 3 h after LPS treatment, lavage fluid from *CCR4*+/+ also contained large numbers of erythrocytes (Figs. 5d, f) which may be indicative of increased vascular permeability or haemorrhaging in these mice. Erythrocytes were absent or markedly reduced in the lavage of *CCR4*-/- mice (Figs. 5c,e). Elevated TNFα production in the *CCR4*+/+ mice may be associated with the increased vascular permeability. It is also possible that the absence of haemorrhaging observed in the *CCR4*-/- mice results from altered platelet function, although at present there is no experimental evidence in support of this hypothesis.
We also looked at the expression of the macrophage (F4/80) and granulocyte (GR1) markers in the peritoneal lavage cells by FACs. Few GR1 positive staining cells were initially detected in the lavage but gradually increased in both *CCR4*+/+ and -/- mice up to 24 h after LPS treatment (Fig. 6a). At this time there was a striking difference in the number of GR1 positive cells present in the CCR4-/- mice compared to *CCR4*+/+ mice. In contrast F4/80 positive cells decreased in both groups of mice with time after LPS treatment. Interestingly in the *CCR4*-/- mice at 24 h there was not only a marked reduction in the total number of F4/80 expressing cells but there was also a decrease in the F4/80 expression level (Figs. 6c,d). These results imply a defect or deficiency in a particular macrophage population expressing F4/80. The F4/80 antigen is an unusual seven transmembrane receptor in that its extracellular domain is composed of EGF domain repeats. Its ligand(s) and function remain unknown but our results suggest that it may be important in the mechanism of LPS resistance in *CCR4*-/- mice.
In view of the differences in the cellular composition of the peritoneal lavage between the CCR4-/- and +/+ mice, we next looked at the effect of LPS administration on the expression of several chemokines which may be involved in the recruitment of inflammatory cells to the peritoneum. MIP-1α, a CC chemokine which has been shown to play an important role in the acute response to LPS is produced by peritoneal macrophages (McKnight A.J. et al, (1996), J. Biol. Chem. 271, 486-489) and in mice, functions as a neutrophil chemoattractant (Van Otteren et al., 1994). Serum MIP-1α levels rose in parallel with TNFα but returned to base line levels by 6 h after LPS treatment (Fig. 7a). Like TNFα, the serum level of MIP-1α in the *CCR4*-/- mice was at least 3 fold lower than that seen in the *CCR4*+/+ mice. Macrophages also produce MIP-2, a neutrophil chemoattractant homologous to IL-8 (Cook D.N. et al., 1995) and the CCR4 ligand, MDC and their expression has previously been shown to be upregulated by LPS. Using semi-quantitative reverse transcriptase PCR of peritoneal lavage cells we observed no significant differences in MIP-2 mRNA expression between the *CCR4*+/+ and *CCR4*-/- mice (Fig. 7b). MDC mRNA expression was similar in both groups of mice at early time points. However at 24 h, the mRNA level fell sharply in the *CCR4*-/- mice but was maintained in the *CCR4*+/+ mice. Although mRNA levels do not necessarily correlate with protein expression, it is possible that the lack of MDC results in altered recruitment of a specific monocyte/macrophage population to the peritoneum, consistent with the results seen by FACs.
Resistance to LPS induced lethality has now been demonstrated in a number of gene targeted mice including MIF (macrophage migration inhibition factor) -/- mice (Rodenburg R.J.T. et al., (1998), J. Leukocyte Biol. 63, 606-611). A possible mechanism of LPS resistance in the CCR4 deleted mice may be due to down regulation of MIF production by peritoneal macrophages. However there was no evidence for this at the level of MIF mRNA as measured by RT-PCR (data not shown).
Whilst a great deal is known about the intracellular events that occur following LPS treatment, relatively little is known about the actual events which occur at the cell surface and the signal transduction mechanism by which LPS induces host cell activation. In the currently accepted model, LPS monomers are catalytically transferred by a lipid exchange molecule LBP (Bozza M.et al., (1999) J.Exp. Med. 189, 341-346), to CD14, a major LPS receptor that lacks a transmembrane domain. The LPS transmembrane coreceptor toll receptor like 4 (Tlr4), is then responsible for the initiation of cellular responses after interaction with the LPS-CD14 complex. The roll of Tlr4 in LPS signalling has been demonstrated following the identification of strains of mice which contained mutations in the Tlr 4 gene rendering them resistant to the lethal effects of LPS. There were no differences in expression of Tlr 4 mRNA in *CCR4*-/- mice compared to *CCR4*+/+ mice in peritoneal lavage cells (data not shown) and LPS resistance was not due to the presence of a mutated Tlr 4 coding sequence in the *CCR4*-/- mice.
Identification of the precise mechanisms of LPS induced cell stimulation is important for our understanding of bacterial pathogenesis and for the development of strategies to protect against gram negative bacterial infection. Targeted deletion of the CCR4 gene reveals an unexpected role for this receptor in LPS induced sepsis. Neutralization of CCR4 either by antibodies or chemokine receptor antagonists may therefore be important therapeutic approaches for the treatment of sepsis.

### Methods

Total RNA was isolated using the Trizol (Gibco BRL) and 1 µg total RNA was reverse transcribed using Superscript (Gibco BRL). One twentieth of the cDNA synthesis reaction was then subjected to 25-40 cycles of PCR using AmpliTaq and PCR primers based on the Genbank database entries for MIP-2 and mMDC. PCR products were analyzed on 1 % agarose gels stained with ethidium bromide and bands migrating at the correct molecular weight were verified by direct sequencing. PCR bands were quantitated using Kodak Digital Science version 1.0 software and are expressed as arbitrary units of mRNA.

### References

Akira S. et al., (1993), Adv. Immunol. 54, 1-78.
Blyth D.I. et al., (1996) Am. J. Resp. Cell Mol. Biol. 14, 425-438.
Bonecchi R. et al, (1998) J. Exp. Med. 187, 129-134.
Bonecchi, R. et al. J. Exp. Med. (in press)
Bozza M.et al., (1999) J.Exp. Med. 189, 341-346.
Chvatchko Y. et al, (1996), J. Exp. Med. 184, 2353-2360.
Conquet F. et al., (1994), Nature 372, 237-243.
Cook D.N. et al., 1995
D'Ambrosio D. et al., 1998
Flory C. M. et al. (1993) Lab. Invest., 69:396-404
Folkesson H. G. et al. (1995), J. Clin. Invest., 96:107-116.
Freudenberg et al., 1986.
Hamelmann E. et al., (1997), Am. J. Respir. Crit. Care Med. 156, 766-775.
Hoogewerf A.J. et al., (1996), Biochem. Biophys. Res. Commun. 218, 337-343.
Imai T. et al., (1997), J. Biol. Chem. 272, 15036-15042.
Imai T. et al., 1999
Imai T., et al. (1998), J. Biol. Chem. 273, 1764-1768.
Lloyd C. M. et al. (1997) J. Exp. Med., 185:1371-1380.
McKnight A.J. et al, (1996), J. Biol. Chem. 271, 486-489.
McMahon A.P et al., (1990) Cell, 62, 1073-1085
Pajkrt D. et al., (1996) Curr. Topics Microbiol. Immunol 216, 119-132.
Perera P-Y. et al., (1998), Infec. Immun. 66, 2562-2569.
Power C.A. et al, (1995) Cytokine 7, 479-482.
Power C.A. et al., (1995), J. Biol. Chem. 270, 19495-19500.
Quereshi S.T. et al., (1999) J.Exp. Med. 189, 615-625.
Rodenburg R.J.T. et al., (1998), J. Leukocyte Biol. 63, 606-611.
Sallusto F. et al, (1998), J. Exp. Med. 187, 875-883.
Shibazaki M. et al. (1996) Infection and Immunity 64, 5290-5294.
Standiford T. J. et al. (1995) J. Immunol., 155:1515-1524.
Standiford T.J.et al., (1995) J.Immunol. 155, 1515-1524.
Van Otteren et al., 1994
Wright S.D. (1999) J.Exp. Med. 189, 605-609.
Yokoi K. I. et al. (1997) Lab. Invest., 76:375-384.

## Claims

1. Use of a CCR4 antagonist together with a pharmaceutically acceptable carrier in the preparation of a pharmaceutical composition for the treatment and/or prevention of septic shock.

2. The use of Claim 1 wherein said CCR4 antagonist is a polypeptide able to bind a specific epitope of CCR4.

3. The use of Claim 1 wherein the CCR4 antagonist is an anti-CCR4 antibody or a fragment thereof.

4. The use of Claim 11 wherein the monoclonal antibody is selected from the group consisting of: a chimeric monoclonal antibody, a humanized monoclonal antibody or fragment thereof.

5. A method for treating and/or preventing septic shock which comprises administering to a patient a therapeutically effective dose of a CCR4 antagonist.

6. Pharmaceutical composition containing a CCR4 antagonist, together with a pharmaceutically acceptable carrier, in the treatment and/or prevention of septic shock.

7. The pharmaceutical composition according to Claim 6, wherein the CCR4 antagonist has the features set out in any one of Claims 1 to 4.
